# EUROPEAN PATENT SPECIFICATION

(11) **EP 1 539 090 B1**
(45) Date of publication and mention of the grant of the patent: **17.09.2008**
(21) Application number: 03791576.6
(22) Date of filing: 16.07.2003
(51) Int. Cl.: A61K 8/81, A61Q 5/06

(54) **NATURAL FEEL POLYMERS**
SICH NATüRLICH ANFüHLENDE POLYMERE
POLYMERES AU TOUCHER NATUREL

(30) Priority: 30.08.2002 US 233838; 29.01.2003 US 353390; 20.02.2003 US 449248 P
(43) Date of publication of application: 15.06.2005
(73) Proprietor: ISP INVESTMENTS INC., Wilmington, Delaware 19801 (US)
(72) Inventor: ULMER, Herbert, 1406 TB Bussum (NL); GILLECE, Timothy, Pompton Plains, NJ 07444 (US); KATIRGIS, John, West Caldwell, NJ 07006 (US)
(74) Representative: Harrison, Susan Joan
(86) International application number: PCT/US2003/021955
(87) International publication number: WO 2004/019858

(56) References cited:
- WO-A-00/19968
- WO-A1-99/67216
- US-A- 5 877 204
- US-A- 5 886 194
- US-A- 5 994 385
- US-A- 6 025 501

## Description

### BACKGROUND OF THE INVENTION

### 1. Field of the Invention

This invention relates to polymers useful in personal care products, and, particularly to hair styling polymers, referred to herein as "natural feel" polymers, which possess the desirable physical attributes of flexibility and a smooth feel, as well as water-solubility and water-resistance.

### 2. Description of the Prior Art

US-A-5 886 194, WO-A- 00/19968, US-6 025 501, US-A-5 994 385 and WO-A-99/67216 disclose maleic acid ester/maleamic acid/maleimide polymers for use in personal care products. The amines used are α-unsubstituted primary amines or alkanol-amines.

Hair styling polymers which feel stiff on hair are rather brittle under a high applied stress; accordingly, these polymers will shatter easily when strained appreciably. On the other hand, highly flexible polymers when used in hair styling will bend under both high and low stress but they are generally considered by the user to be too soft for desirable hair styling.

Accordingly, it is an object of this invention to provide polymers which have the desirable attributes of stiffness and flexibility, and have a strong affinity for hair imparting a natural feel for the user, and are also water-soluble and water-resistant.

Another object herein is to provide natural feel polymers which can be easily removed from a substrate such as hair or skin, or a textile fiber, by simple washing.

These and other objects and features of the invention will be made apparent from the following description thereof.

### SUMMARY OF THE INVENTION

Natural feel polymers, referred to as "A" polymer, have defined amounts of repeat units of (a) (b) maleamic acid, and (c), as shown below:

### NATURAL FEEL POLYMERS

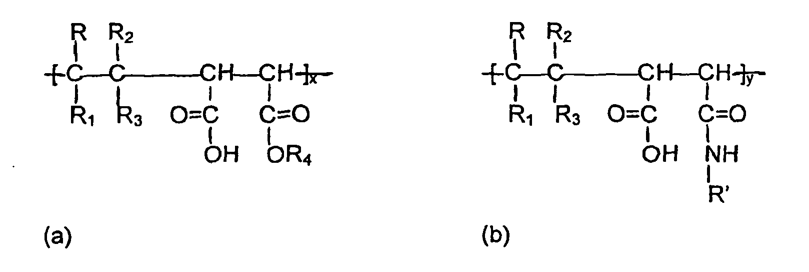

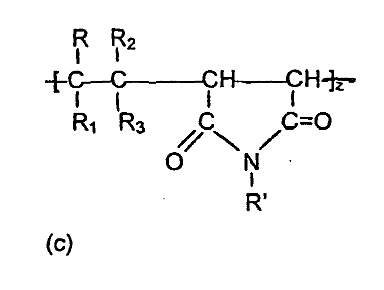

where:
R, R₁, R₂ and R₃ are selected from H, alkyl, alkoxy, cycloalkyl, aryl, ester, acid, hydroxy, hydroxyalkyl, amido, fluoro, halo and silyl, and R₄ is H or alkyl; and
R' is a polyoxyalkylene amine of formula 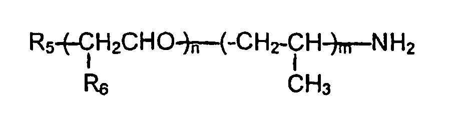 where R₅ and R₆ are selected from H and alkyl; e.g. R₅ is CH₃ and R₆ is H; and R₅ is CH₃ and R₆ is CH₃; and n and m are integers from 1-50; e.g. n = 32 and m = 10.
x, y and z are present, in mole %, of 0-99.9, 0-50 and 0.1-100, respectively; preferably 0-50, 0-5 and 50-100.

Mixtures of "A" polymer and "B", a linear or cross-linked acrylic acid polymer, produces useful polymers with advantageous properties.

### DETAILED DESCRIPTION OF THE INVENTION

The natural feel polymers (A) of the present invention are particularly characterized by repeat units which contain an abundance (by weight) of polyether amine derivatizing groups which can hydrogen-bond with itself or other repeat units in the polymer to form an intra- or inter-molecular polymer resulting in a pseudo-network polymer. This polymer thus acts as if it is crosslinked. Cohesion between such hydrogen-bonded molecules provides the polymer with water-resistance, but also with water solubility because, once the polymer is flooded with water, it will admit sufficient amount of water for solubilization. The polymers show good adhesion to natural substrates but can be removed easily if desired. Some polyether derivatizers may also crystallize upon dry-down, resulting in enhanced properties such as water resistance.

Representative structural components of the natural feel polymers of the invention are given below.

### Reactants for Natural Feel Polymers

### Polymer Backbone

### Monomer-Maleic Anhydride Copolymer

Alkyl vinyl ether-maleic anhydride copolymer, e.g. methyl vinyl ether-maleic anhydride copolymer, or isobutyl vinyl ether-maleic anhydride copolymer; and derivatives thereof, including alpha-olefin-maleic anhydride copolymer, e.g. ethylene-maleic anhydride copolymer, or isobutylene-maleic anhydride copolymer; styrene-maleic anhydride copolymer, etc. The polyoxyalkylene amines are obtainable as Jeffamine^{®} M Monoamines (Huntsman Corp), with various molecular weights and ethylene oxide (EO)/propylene oxide (PO) ratios. These amines are present to provide natural feel properties in the polymer, i.e. softness and flexibility, as well as the desired adhesive/cohesive balance.

Another feature of the invention is the provision of products which are made by mixing the natural feel polymers, (A), with (B) a linear or crosslinked acrylic acid polymer, e.g. Carbopol^{®}, preferably which is neutralized before mixing with (A). The result of mixing (A) and (B) is a complexed, synergistic product particularly suitable for hair care application because it has high humidity curt retention, and has an increased solution viscosity, as compared to (A) or (B) alone. The viscosity of the product can be predetermined by the relative amounts of (a), (b) and (c) in polymer (A). For example, 100 mole % in the (c) repeat unit will provide an opaque product, while dilution of the polymer with more (a) repeat units will form a more desirable clear, and less viscous product, upon mixing with (B).

The polymers strongly interact and upon dry down result in films with increased toughness and cohesiveness than individual polymer systems..

When these synergistic systems are used in a personal care hair styling application the resultant formulations have improved high humidity and curl retention (HHCR) when compared to similar formulations containing the individual polymers alone.

The degree of complexation between (A) and (B) can be predetermined by adjusting the mole ratio of maleimide in (A) to the carboxylic units in (B). Complexation is strongest for products in which the maleimide:carboxylic mole ratio approaches 1:1.

However, complexation is not as pH sensitive as typical acid-base complexed systems, e.g. PVP and acrylic acid; in fact, complexation can occur at a neutral pH. Thus personal care formulations at or around a neutral pH still possess synergistic complexation complexation of (A) and (B), with its resultant desirable properties and physical attributes.

The invention will now be described with reference to the following examples.

### EXAMPLE 1

### Preparation of Polymer (A)

The following were charged into a 2-liter, stainless steel high pressure reactor.

| | |
|---|---|
| P(maleic anhydride/isobutene) (Man) | 72.94 g |
| 3-(dimethylamino) propylamine (50 mol % based on Man) | 24.17 g |
| Jeffamine^{®} M-2070 (2 mol % based on Man) (M.W. 2,000, 70/30 EO/PO) (water soluble) | 20.73 g |
| Triethylamine (43 mol % based on Man) (Neutralizer) | 20.59 g |
| Methanol | 257.07 g |

The reactor was sealed, purged 3 times with N₂ gas, and heating was initiated according to the following heating profile.
- Ambient → 90°C, 1½ hr.
- 90°C → 90°C, 2 hr.
- 90°C → 130°C, 1½ hr.
- 130°C → 130°C, 8 hr.
- 130°C → 35°C, 1 hr.

At the end of the heating cycle, the polymer product was obtained as a lightly viscous, yellow, clear solution; then it was flooded with water to give a viscous, hazy, yellow-colored solution. The solution was mixed with neutralized Carbopol^{®} in an amount present in a typical styling gel formulation. A thick, clear gel was obtained after an hour. The gel formulation was applied to hair and the resultant film was stressed. The film showed a natural feel, combining firm and flexible characteristics, water-resistance and water-solubility, and excellent high humidity curl retention.

### Mixing Polymer (A) with Carboxylic Acid Containing Polymer (B)

The polymer solution (A) was mixed with neutralized Carbopol^{®}, a crosslinked acrylic acid polymer, in amounts of 2 and 0.5 wt.%, respectively, and in an amount present in a typical styling gel formulation. A thick gel having a viscosity greater than (A) or (B) was obtained after an hour. The gel formulation was applied to hair and the resultant film was stressed. The film showed a natural feel, combining firm and flexible characteristics, water-resistance and water-solubility, and excellent high humidity curl retention.

### EXAMPLE 2

The following reactants were added to a 2-liter pressure reactor:

| | |
|---|---|
| Poly(isobutylene/maleic anhydride) | 119.22 g |
| Dimethylaminopropylamine | 39.51 g |
| Jeffamine M-2005 (M.W. 2,000, 5/95 EO/PO (water-insoluble) | 42.35 g |
| Jeffamine M-2070 (M.W. 2,000, 70/30 EO/PO (water-soluble) | 42.35 g |
| Triethylamine | 31.30 g |
| Ethanol | 510.20 g |

The reactor was sealed and purged with an inert gas. The following heating profile was initiated:

| | | |
|---|---|---|
| Heat to 125°C | - | 4 hours |
| Hold at 125°C | - | 12 hours |
| Cool to 35°C | - | 1 hour |

After the heating profile was complete, the polymer solution was discharged from the reactor. The resultant material was a viscous, clear yellow solution. This material was laid down as a film and allowed to dry. A non-brittle film resulted which was water soluble. Exchanging water for ethanol gave a water-based solution having similar properties to the ethanol-based material.

The carboxylic acid-containing polymer can be formed as above.

### EXAMPLE 3

The following reactants were added to a 2-liter pressure reactor:

| | |
|---|---|
| Poly(isobutylene/maleic anhydride) | 119.22 g |
| Dimethylaminopropylamine | 39.51 g |
| Jeffamine M-2005 | 84.70 g |
| Triethylamine | 31.30 g |
| Ethanol | 510.20 g |

The reactor was sealed and purged with an inert gas. The following heating profile was initiated:

| | | |
|---|---|---|
| Heat to 125°C | - | 4 hours |
| Hold at 125°C | - | 12 hours |
| Cool to 35°C | - | 1 hour |

After the heating profile was complete, the polymer solution was discharged from the reactor. The resultant product was a viscous, clear yellow solution. This material was laid down as a film and allowed to dry. A non-brittle film resulted. Exchanging with water gave a water-based solution having similar properties to the ethanol-based material.

The carboxylic acid polymer can be reacted as above, if desired, to produce new and useful polymers, as described in Example 1.

### EXAMPLE 4

The following reactants were added to a 2-liter pressure reactor:

| | |
|---|---|
| Poly(isobutylene/maleic anhydride) | 119.22 g |
| Dimethylaminopropylamine | 39.51 g |
| Jeffamine M-2005 | 84.70 g |
| Jeffamine M-2070 | 33.90 g |
| Triethylamine | 31.30 g |
| Ethanol | 573.97 g |

The reactor was sealed and purged with an inert gas. The following heating profile was initiated:

| | | |
|---|---|---|
| Heat to 125°C | - | 4 hours |
| Hold at 125°C | - | 12 hours |
| Cool to 35°C | - | 1 hour |

After the heating profile was complete, the polymer solution was discharged from the reactor. The resultant material was a viscous clear yellow solution. This material, when laid down as a film and allowed to dry, resulted in a flexible film. This same material can be exchanged with water to give a water-based solution having similar properties to the ethanol-based material.

The carboxylic acid-containing polymer can be formed as in Example 1.

### EXAMPLE 5

The following reactants were added to a 2-liter pressure reactor:

| | |
|---|---|
| Poly(isobutylene/maleic anhydride) | 119.22 g |
| Dimethylaminopropylamine | 39.51 g |
| Jeffamine M-2005 | 101.70 g |
| Jeffamine M-2070 | 84.70 g |
| Triethylamine | 31.30 g |
| Ethanol | 699.08 g |

The reactor was sealed and purged with an inert gas. The following heating profile was initiated:

| | | |
|---|---|---|
| Heat to 125°C | - | 4 hours |
| Hold at 125°C | - | 12 hours |
| Cool to 35°C | - | 1 hour |

After the heating profile was complete, the polymer solution was discharged from the reactor. The resultant material was a viscous clear yellow solution. This material, when laid down as a film and allowed to dry, resulted in a very-flexible film. This same material can be exchanged with water to give a water-based solution having similar properties to the ethanol-based material.

The carboxylic acid-containing polymer can be formed as in Example 1.

The natural feel polymers of Examples 1-5 were formulated into typical hair care products designed for use in the modes of styling, mousse, gel and spray hair care products. These products performed well in practice giving the user the advantages of the natural feel polymers therein, particularly a firm and flexible characteristic, water-resistance and water-solubility, and excellent high humidity curl retention.

More particularly, the compositions described herein are useful in products for personal care, including, but not limited to, gels, lotions, glazes, glues, mousses, sprays, fixatives, shampoos, conditioners, 2n1 shampoos, temporary hair dyes, semi- permanent hair dyes, permanent hair dyes, straighteners, permanent waves, relaxers, creams, putties, waxes and pomades. The compositions can be used alone or in combination with anionic, nonionic and cationic hair styling polymers, thickeners, film formers, surfactants, reducing agents, oxidizers and other ingredients typically found in personal care products. Specific examples follow:
Gels: Hair and/or skin care compositions wherein the compositions comprise an aqueous or hydroalcoholic gel. Gels can be in the form of spray gels, fluid gels, tube gels and thick viscous tub gels. The compositions can be used preferably at use levels of 0.1 -10% by weight in anionic, nonionic or cationic gallants (clear, translucent or opaque), or combinations thereof, such gellants preferably being present in amounts of 0.1-5% by weight.

Anionic gellants include, but are not limited to, carbomer, AcrylateslC10-30 Alkyl Acrylate Crosspolymer, Acrylates Copolymer, Acrylates/Beheneth-25 Methacrylate Copolymer, Acylates/Steareth-20 Methacrylate Copolymer, PVM/MA Decadiene Crosspolymer, Xanthan Gum, sodium polyacrylate, polyacrylamide, copolymers of sodium acrylates, and copolymers of polyacryalmide.

Nonionic gellants include, but are not limited to, guar and their derivatives and celluloses and their derivatives. Examples are hydroxyethyl cellulose, hydroxypropyl cellulose, hydroxymethyl cellulose, hydroxypropyl guar.

Cationic thickeners include, but are not limited to, Polyqaternium 32 (and) mineral oil, and Polyquaternium 37 (and) mineral oil (and) PPG-1 Trideceth-6.

Hair and skin care gel formulations with the compositions using crosslinked homopolymers of acrylic acid, e.g., carbomer and/or Acrylates/C10-30 Alkyl Acrylate Crosspolymer as the gellant result in synergistic performance in moisture resistance. In particular, hair styling gels with the above listed combinations show synergistic high humidity resistance on hair.

Complexation of the compositions with carbomer and/or Acrylates/C10-30 Alkyl Acrylate Crosspolymer results in clear films upon draw down. The resultant viscosity, yield value and suspension capabilities are unaffected or increased by the addition of such compositions into the gellant.

### Formulation Examples:

### Hair Styling Gel Formulation 1

Water- QS to 100%
Carbomer - 0.5%
Triethanolamine(99%) - 0.5%
Isobutylene/dimethylaminopropylmaleimide/ethoxylated maleimide/maleic
acid copolymer (30%)- 6.66
Benzophenone-4 - 0.05
Disodium EDTA - 0.10
Triethanolamine(99%) - adjust to pH 7
Propylene Glycol (and) diazolidinyl Urea (and) lodopropynyl butylcarbamate - 0.5

### Hair Styling Gel Formulation 2

Water - QS to 100%
Isoceteth-20 - 0.5
Isobutylene/dimethylaminopropylmaleimide/ethoxylated maleimide/maleic
acid copolymer(30%) - 6.66
Aminomethyl propanol - 0.25%
Acrylates/Beheneth-25 Methacrylate Copolymer(20%) - 4.7
Propylene Glycol (and) Diazolidinyl Urea (and) lodopropynyl Butylcarbamate -0.5

### Hair Styling Cream

Water - QS to 100%
Sodium Polyacrylate(and) Hydrogenated polydecene (and) Trideceth-6 - 2.0%
Isobutylene/dimethylaminopropylmaleimide/ethoxylated maleimide/maleic
acid copolymer (30%) - 6.7%
Glycerin - 2.0%
Cetyl PEG/PPG-15/15 Butyl Ether Dimethicone -1.0%
Propylene Glycol (and) Diazolidinyl Urea (and) lodopropynyl Butylcarbamate - 0.5%

Mousses: The compositions can be incorporated into aerosol and non-aerosol hair and skin mousse formulations, as well as spray mousses which utilize an aerosol valve with a dip tube and a mechanical break-up actuator to deliver an atomized spray foam. They are also compatible in aerosol and non-aerosol shave foam applications. Preferred use levels of the compositions are 0.1-10.0% by weight.

Shampoos and Body Washes: The compositions described are compatible with anionic, amphoteric, cationic and nonionic surfactants. The compositions can be incorporated into cleansing formulations for hair and body. The compositions described can be used preferably at use levels of 0.1 to 10% by weight with anionic, amphoteric, cationic, and nonionic surfactants, or combinations thereof, such surfactants preferably being present in amounts of 0.1 to 20% by weight.

### Clear Shampoo Formulation Example:

Water - QS to 100%
Isobutylene/dimethylaminopropylmaleimide/ethoxylated maleimide/maleic
acid copolymer - 5.0
Cocamidopropyl Betaine (34.5%) -10.00%
Cocoamphodiacetate (50%) - 5.00%
Sodium Laureth Sulfate (25.6) -17.5%
Ammonium Lauryl Sulfate (29.2%) -17.5%
Propylene Glycol (and) Diazolidinyl Urea (and) lodopropynyl Butylcarbamate -0.30
Citric Acid - 0.25%

Oil-in-Water Emulsions and Hair Conditioners (including both leave-in and rinse-out): The compositions can be incorporated in hair and skin oil-in-water emulsions. In hair conditioners, the compositions described are compatible with quaternary ammonium compounds. The use level of surfactants/emulsifiers preferably is from 0.1 to 10% by weight.

### Conditioner Formulation Example

### Phase A:

Water - QS to 100%
Citric Acid - 0.25
Hydroxyethyl cellulose - 0.5%
Disodium EDTA - 0.1%

### Phase B:

Cetearyl Alcohol - 4.0
Steareth-10 -1.0
Glyceryl Stearate (and) PEG-100 Stearate - 2.5%
Dicetyldimonium Chloride(68.2%) - 2.0%

### Phase C:

Isobutylene/dimethylaminopropylmaleimide/ethoxylated maleimide/maleic acid copolymer(30%) - 6.6
Propylene Glycol (and) Diazolidinyl Urea (and) lodopropynyl Butylcarbamate -0.5
Wheat Amino Acid - 0.5

Process: Heat Phase A and B separately to 75C. Add Phase B to Phase A with agitation. Mix until uniform and cool to 45C. Continue mixing and add ingredients in Phase C one at a time with mixing until homogenous.

Oxidative Hair Dyes: The compositions may be incorporated in oxidative hair dye formulations including semi-permanent and permanent hair dye products, preferably at use levels of 0.1-10% by weight.

### Formulation Example:

Water-QS to 100%
Oleic Acid -5%
C11-15Pareth-9 - 3%
Isobutylene/dimethylaminopropylmaleimide%thoxylated maleimide/maleic
acid copolymer(30%) - 3.0
Ammonium hydroxide -2%
Steareth-21 - 2%
Propylene Glycol - 2%
Cetyl alcohol - 2%
PEG150/Stearyl/SMDI Copolymer-3%
Stearyl Alcohol - 1%
Sodium Sulfite - 1%
Iron Oxides - 0.5%
Mica - 0.2%
1-Naphitol - 0.2%
P-phenylendiamine - 0.2%
Titanium Dioxide - 0.1 %

Relaxers and Permanent Waves: The compositions can be used in relaxer and permanent wave formulations preferably in amounts of 0.1% - 10% by weight. They may be combined with hair reducing agents, including, but not limited to, ammonium thioglycolate, guanidine hydroxide, sodium bisulfite and the like. The compositions can also be incorporated in the flow lotion (treatment phase before oxidation/hardening of the hair) or in the neutralizer/oxidizer phase.

### Formulation Example:

Crème Hair Relaxer Base: To be mixed with Guanidine Carbonate Activator Solution
Water-QS to 100%
Petrolatum - 10%
Paraffin Wax - 8%
Mineral Oil - 6%
Isobutylene/dimethylaminopropylmaleimide/ethoxylated maleimide/maleic
acid copolymer (30%) - 5.0
Cetearyl Alcohol - 3%
Calcium Hydroxide - 3%
Polysorbate 80-2%
Laneth-15-2%
PEG-75 Lanolin Oil -1%
Cocamphodipropionate -1 %

Hair Sprays: The compositions can be incorporated in hair sprays, both non-aerosol and aerosol, preferably at use levels of 0.1-10% by weight. Aerosol hair sprays can include up to 60% hydrocarbon, 70% dimethyl ether, 50% Hydrofluorocarbon 152a, or combinations thereof. Hair spray formulations include, but are not limited to, alcohol-free pump hair spray, 55%-95% VOC pump and aerosol hair sprays.

### Formulation Example:

Clear Alcohol Free Pump Hair Spray
Water - QS to 100%
Sodium Lauryl Sulfate (25%) - 0.25%
isobutylene/dimethylaminopropylmaleimide/ethoxylated maleimide/maleic acid copolymer(30%) - 13.33
Propylene Glycol (and) Diazolidinyl Urea (and) lodopropynyl Butylcarbamate - 0.5%
Pump - Calmar Mark VI WL-31

Personal Care Applications: The compositions can be blended with anionic, nonionic and cationic hair styling polymers, thickeners, and film formers; and with anionic, nonionic and cationic surfactants. Clarity in water is increased with low levels of charged surfactants (0.1-2% by weight).

Performance on Hair: The compositions can be formulated into bodifying leave-on or rinse-off hair preparations. They also can be formulated into flexible hold styling products which render smooth continuous films on hair that have strength and will bend under both high and low stress.

Skin Care Applications: The compositions can be used as a film former (a) for the enhancement of antiperspirants to either increase overall wetness protection or reduction in amount of conventional AP active while holding equivalent efficacy, (b) to increase the substantivity of a deodorant active for better and longer acting deodorancy, (c) in an anti-bacterial liquid hand soap to increase efficacy and for longer lasting claim, (d) for holding products on skin, and (e) to increase contact time of a therapeutic skin products containing actives, including, but not limited to, Betulin, vitamin E, A, C, ceramides, Allantoin, lycopenes, bisabolol, retinol, etc.

The compositions can be used in make-up products, (foundation, mascara, bronzers, eyeliners) for film formation, improved wear resistance and pigment dispersion. They can also be used in mascaras for curl retention.

## Claims

1. A natural feel polymer, "A", which shows an advantageous blend of stiffness and flexibility, a strong affinity to natural fibers, water-solubility and water-resistance, **characterized** structurally by repeat units of (a), (b) and (c) of the formulas: 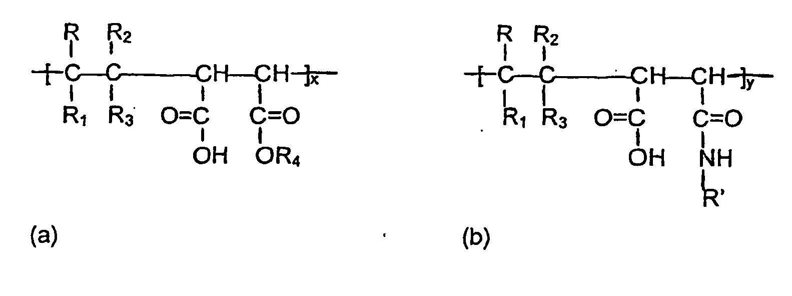 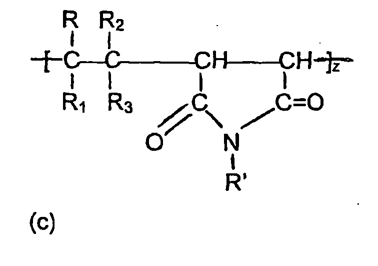 where:
R, R₁, R₂ and R₃ are selected from H, alkyl, alkoxy, cycloalkyl, aryl, ester, acid, hydroxy, hydroxyalkyl, amido, fluoro, halo and silyl, and R₄ is H or alkyl; and
R' is a polyoxyalkylene amine of formula 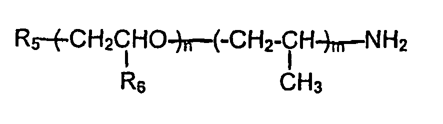 where R₅ and R₆ are selected from H and alkyl; and n and m are integers from 1-50; wherein:
x, y and z are present, in mole %, of 0-99.9, 0-50 and 0.1-100, respectively.

2. The product of mixing the natural feel polymer (A) according to claim 1 with (B), a linear or cross-linked acrylic acid polymer.

3. A natural feel polymer according to claim 1 wherein R₅ is CH₃ and R₆ is H.

4. A natural feel polymer according to claim 1 wherein both R₅ and R₆ are CH₃.

5. A natural feel polymer according to claim 1 wherein n = 32 and m=10.

6. A natural feel polymer according to claims 1 or 2 wherein, in (a), said monomer is an α-olefin.

7. A natural feel polymer according to claim 2 wherein (B) is a crosslinked acrylic acid polymer.

8. A polymer according to claim 2 wherein (B) is a linear acrylic acid containing polymer.

9. A polymer according to claim 2 wherein (B) is a neutralised acrylic acid containing polymer.

10. A personal care formulation including the natural feel polymer of claims 1 or 2.

11. A personal care formulation according to claim 10 which is a hair care product.

12. A personal care formulation according to claim 10 which is a shampoo, body wash, conditioner, relaxer, permanent wave, leave-on or rinse-off hair care formulation.

13. A hair care formulation according to claim 10 which is a styling, mousse, gel or spray hair care formulation.

14. A hair care formulation according to claim 10 which is an oxidative hair dye formulation, permanent wave, bleach or other treatment product.

15. A personal care formulation according to claim 10 which is a skin care formulation.

16. A product according to claim 2 wherein the mole ratio of maleimide in (A) to the carboxylic units in (B) is about 1:1.

17. A personal care formulation according to claim 10 which is a make-up product.

## Patentansprüche

1. Sich natürlich anfühlendes Polymer "A", das eine vorteilhafte Mischung von Steifheit und Flexibilität, eine starke Ähnlichkeit zu natürlichen Fasern, Wasserlöslichkeit und Wasserbeständigkeit aufweist und strukturell durch Grundeinheiten (a), (b) und (c) mit folgenden Formeln **gekennzeichnet** ist: 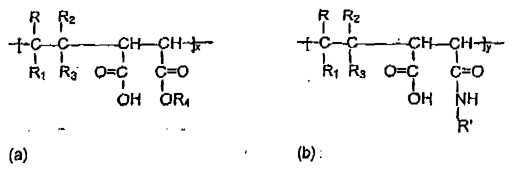 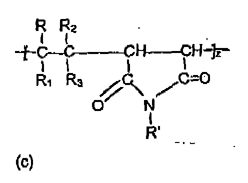 worin:
R, R₁, R₂ und R₃ aus H, Alkyl, Alkoxy, Cycloalkyl, Aryl, Ester, Säure, Hydroxy, Hydroxyalkyl, Amido, Fluor, Halogen und Silyl ausgewählt sind und R₄ H oder Alkyl ist und
R' ein Polyoxyalkylenamin folgender Formel ist: 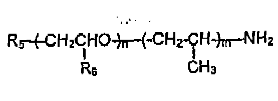
worin R₅ und R₆ aus H und Alkyl ausgewählt sind und n und m ganze Zahlen von 1 bis 50 sind; worin:
x, y und z in einer Menge von 0 bis 99,9; 0 bis 50 bzw. 0,1 bis 100 Mol-% vorhanden sind.

2. Produkt des Mischens des sich natürlich anfühlenden Polymers (A) nach Anspruch 1 mit (B), einem unverzweigten oder vernetzten Acrylsäurepolymer.

3. Sich natürlich anfühlendes Polymer nach Anspruch 1, worin R₅ = CH₃ und R₆ = H ist.

4. Sich natürlich anfühlendes Polymer nach Anspruch 1, worin sowohl R₅ als auch R₆ CH₃ sind.

5. Sich natürlich anfühlendes Polymer nach Anspruch 1, worin n = 32 und m = 10 ist.

6. Sich natürlich anfühlendes Polymer nach Anspruch 1 oder 2, worin das Monomer in (a) ein α-Olefin ist.

7. Sich natürlich anfühlendes Polymer nach Anspruch 2, worin (B) ein vernetztes Acrylsäurepolymer ist.

8. Polymer nach Anspruch 2, worin (B) ein unverzweigtes acrylsäurehältiges Polymer ist.

9. Polymer nach Anspruch 2, worin (B) ein Polymer ist, das neutralisierte Acrylsäure enthält.

10. Körperpflegeformulierung, umfassend das sich natürlich anfühlende Polymer nach Anspruch 1 oder 2.

11. Körperpflegeformulierung nach Anspruch 10, die ein Haarpflegeprodukt ist.

12. Körperpflegeformulierung nach Anspruch 10, die ein Shampoo, ein Duschgel, eine Haarspülung, ein Haarglättungsmittel, ein Dauerwellenmittel, eine Haarpflegeformulierung ohne Auswaschen oder eine Haarpflegeformulierung, die ausgewaschen werden muss, ist.

13. Haarpflegeformulierung nach Anspruch 10, die eine Styling-, Mousse-, Gel- oder Spray-Haarpflegeformulierung ist.

14. Haarpflegeformulierung nach Anspruch 10, die eine oxidative Haarfärbeformulierung, ein Dauerwellenmittel, ein Bleichmittel oder ein anderes Behandlungsprodukt ist.

15. Körperpflegeformulierung nach Anspruch 10, die eine Hautpflegeformulierung ist.

16. Produkt nach Anspruch 2, worin das Molverhältnis des Maleinimids in (A) zu den Carboxyleinheiten in (B) etwa 1:1 beträgt.

17. Körperpflegeformulierung nach Anspruch 10, die ein Make-up-Produkt ist.

## Revendications

1. Polymère au toucher naturel, "A", qui montre un mélange avantageux de raideur et flexibilité, une forte affinité pour les fibres naturelles, une solubilité dans l'eau et une résistance à l'eau, **caractérisé** structurellement par des unités récurrentes de (a), (b) et (c) des formules: 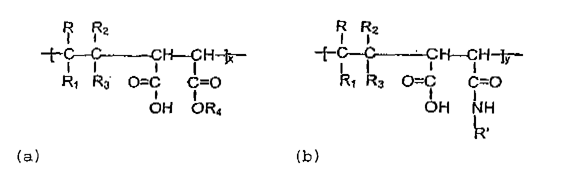 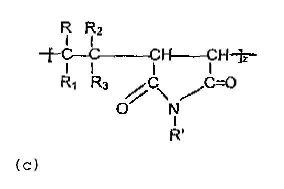 où:
R, R₁, R₂ et R₃ sont sélectionnés parmi H, alkyle, alcoxy, cycloalkyle, aryle, ester, acide hydroxy, hydroxyalkyle, amido, fluoro, halo et silyle, et R₄ est H ou alkyle; et
R' est une polyoxyalkylène amine de formule 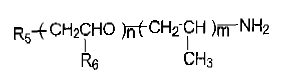
où R₅ et R₆ sont sélectionnés parmi H et alkyle; et n et m sont des entiers de 1 à 50;
où x, y et z sont présents, en pour cent en moles de 0-99,9, 0-50 et 0,1-100 respectivement.

2. Produit du mélange du polymère au toucher naturel A selon la revendication 1 avec (B), un polymère d'acide acrylique linéaire ou réticulé.

3. Polymère au toucher naturel selon la revendication 1 où R₅ est CH₃ et R₆ est H.

4. Polymère au toucher naturel selon la revendication 1 où R₅ et R₆ sont tous deux CH₃.

5. Polymère au toucher naturel selon la revendication 1 où n=32 et m=10.

6. Polymère au toucher naturel selon les revendications 1 ou 2 où, dans (a), ledit monomère est une α-oléfine.

7. Polymère au toucher naturel selon la revendication 2 où (B) est un polymère d'acide acrylique réticulé.

8. Polymère selon la revendication 2 où (B) est un polymère linéaire contenant de l'acide acrylique.

9. Polymère selon la revendication 2, où (B) est un polymère neutralisé contenant de l'acide acrylique.

10. Formulation de soins personnels comprenant le polymère au toucher naturel des revendications 1 ou 2.

11. Formulation de soins personnels selon la revendication 10 qui est un produit de soins pour les cheveux.

12. Formulation de soins personnels selon la revendication 10 qui est un shampooing, un savon corporel, un conditionneur, un détendeur, une onde permanente, une formulation de soins pour les cheveux avec ou sans rinçage.

13. Formulation de soins pour les cheveux selon la revendication 10 qui est une formulation de mise en forme, en mousse, gel ou spray de soins pour les cheveux.

14. Formulation de soins pour les cheveux selon la revendication 10 qui est une formulation de teinture des cheveux par oxydation, une onde permanente, un blanchiment ou autre produit de traitement.

15. Formulation de soins personnels selon la revendication 10 qui est une formulation de soins pour la peau.

16. Produit selon la revendication 2 où le rapport en moles du maléimide dans (A) aux unités carboxyliques dans (B) est d'environ 1:1.

17. Formulation de soins personnels selon la revendication 10 qui est un produit de maquillage.
